# EUROPEAN PATENT SPECIFICATION

(11) **EP 1 011 582 B1**
(45) Date of publication and mention of the grant of the patent: **02.10.2002**
(21) Application number: 97954620.7
(22) Date of filing: 22.12.1997
(51) Int. Cl.: A61F 13/56

(54) **ABSORBENT ARTICLE HAVING DRAWSTRING MEMBERS**
ABSORBIERENDER ARTIKEL MIT ZUGKORDEL
ARTICLE ABSORBANT A CORDONS DE SERRAGE

(30) Priority: 31.12.1996 US 777849; 24.02.1997 US 777849
(43) Date of publication of application: 28.06.2000
(73) Proprietor: THE PROCTER & GAMBLE COMPANY, Cincinnati, Ohio 45202 (US)
(72) Inventor: ROE, Donald, Carroll, West Chester, OH 45069 (US); FISHER, Constance, Lee, Cincinnati, OH 45246 (US); BERGMAN, Carl, Louis, Loveland, OH 45140 (US); FLENDER, Gregg, Allen, Quincy, MA 02169 (US)
(74) Representative: Kohol, Sonia
(86) International application number: US9723812
(87) International publication number: WO98029080

(56) References cited:
- FR-A- 2 425 205
- GB-A- 665 724
- GB-A- 2 294 865
- US-A- 4 585 447

## Description

### FIELD OF THE INVENTION

The present invention provides an absorbent article comprising drawstring members which provide enhanced fit of the absorbent article about a wearer. The drawstring members extend along the longitudinal edges of the absorbent article and are used to adjust the tension of the article about the legs and/or torso of a wearer.

### BACKGROUND OF THE INVENTION

The need to provide better fit in an absorbent article to a wearer has been well documented. In articles such as diapers and incontinent devices, fastening systems, elastic leg cuffs, elastic waistbands and the like have been developed to provide closer, yet dynamic fit of an absorbent article to a user. For Example, U.S. Patent No. 3,848,594 issued to Buell on November 19, 1974 teaches tape fastening systems and U.S. Patent No. 5,058,247 issued to Thomas on October 22, 1991 teaches hook and loop fastening systems. The use of such fastening systems has provided diaperers with the freedom to adjust and re-adjust diapers, etc. about a wearer as needed.

In another example, U.S. Patent No. 3,860,003 issued to Buell on January 14, 1975 teaches leg cuffs that have been provided to elastically fit about a user's thighs. A user, especially a baby, may position itself in awkward positions necessitating dynamic fit of the diaper to a baby throughout these positions. Additionally, elastic waist bands have worked to provide dynamic, yet snug fit of a diaper about a wearer's waist.

All of these methods have worked well, however each has been structurally independent of one-another and out of the range of adjustment to a diaperer. Furthermore, a diaperer, until now, has been unable to tighten or loosen the elastic leg cuffs and waist bands because of their construction. The leg cuffs and waistbands mentioned above have been constructed such that they are out of reach and unalterable to a user. Manufacturers have set the tension in the leg cuffs and waistbands and then placed each out of the reach of a diaperer.

One opportunity to dynamically fit a diaper about a wearer is taught in U.K. Patent Application 2,001,236 (Raymond Pigneul). In this publication, Pigneul provides tapes, i.e., drawstrings, along each longitudinal side edge of the diaper. These tapes are housed within chambers or folds formed by the diaper chassis. The tapes are anchored at or near both end edges. At one end of the diaper, two openings exist whereby portions of the tapes forming loops protrude therefrom. Once the diaper is fitted on the wearer, the tapes are pulled about the diaper chassis by their loops. These loops are then secured to an anchoring element which is located on the backsheet or impermeable sheet of the chassis. Pigneul's tapes serve to not only create a tightness of his diaper to a user but also secure the diaper to a user without the use of any adhesives.

FR-A-2 425 205 discloses a diaper having drawstrings associated with each longitudinal edge. The drawstrings can be pulled and tied in a knot to secure and tighter the leg openings of the diaper where worn.

Therefore, it is an object of the invention herein to provide drawstrings for the purpose of establishing better fit of a diaper about a user's legs which can function independently of a fastening system.

It is an additional object of the invention herein to provide drawstrings for the purpose of establishing better fit of a diaper through a user's crotch region.

It is a further object of the invention herein to provide drawstrings which can be bonded only at one end of the diaper chassis.

Furthermore it is an object of the invention herein to provide adhesive and/or non-adhesive securing means on the drawstrings for securement of the drawstrings to the diaper chassis.

It is an additional object herein to provide securement of the drawstrings to the diaper chassis with or without a secondary fastening element, e.g., a receiving zone for tapes and/or hooks.

It is a further object herein to provide an embodiment whereby an elastic waist element is in structural and functional cooperation with the drawstrings.

These and other objects of the present invention will be more readily apparent when considered in reference to the following description and when taken in conjunction with the accompanying drawings.

### SUMMARY OF THE INVENTION

Accordingly, the present invention provides an absorbent article comprising a chassis assembly having a topsheet, a backsheet having an inner surface and an outer surface joined to the topsheet, and an absorbent core located between the topsheet and the backsheet. The chassis assembly further comprises a front region, a crotch region adjacent to the front region on one side of the crotch region, a rear region adjacent to the crotch region on an opposing side of the crotch region, a longitudinal axis, a transverse axis, a pair of end edges, a pair of longitudinal edges, and a fastening means whereby the absorbent article is fastened about a wearer. A chamber is positioned adjacent to each longitudinal edge and extends from the front region to the rear region. Each chamber is substantially enclosed and has an end opening in the rear region. A drawstring member extends through each of the chambers, each drawstring member comprising a first end securely fastened to the chassis assembly within the front region and a second end extending outboard of the end opening in the rear region. In another embodiment herein, at least one drawstring and drawstring chamber are positioned substantially parallel to the longitudinal centerline and away from the longitudinal edges. This is to provide a fit means through a user's crotch region.

Preferably, the second ends of the drawstring members of each embodiment herein comprises at least one fastening element for securement of the tension applied in each drawstring member. The fastening elements are selected from the group consisting of hooks, loops, adhesive tapes and cohesives. To complement the fastening elements, the outer surface of the backsheet may comprise one or more receiving elements for connection with the fastening elements. These receiving elements are selected from the group consisting of hooks, loops, and fastening surfaces.

For each embodiment of the present invention, the drawstring members may comprise at least one elastic component. Elastic components are selected from the group consisting of rubber elastics, synthetic rubber elastics, elastomeric foams, structural elastic-like film webs and/or elastomeric films. The drawstring members may also comprise at least one inelastic component. Inelastic components are selected from the group consisting of nonwovens, films, laminates, foams, highloft nonwovens, string, twine, and/or braided rope.

In an alternative embodiment herein, the drawstring chambers are substantially enclosed and have their end openings in the front region rather than the rear region. Furthermore, the drawstring members are securely fastened at their first ends within the rear region and their second ends extend outboard of the chamber openings in the front region.

In another alternative embodiment herein, the chassis assembly comprises a pair of ear members that are positioned adjacent to the end edge in the rear region and extend outboard from the longitudinal edges of the absorbent chassis. Each ear member comprises a grasping zone and a stretch zone. At least one drawstring chamber is positioned adjacent to each longitudinal edge and extends from the ear member in the rear region to the front region. Each chamber is enclosed and a drawstring member extends through each of the chambers. Each drawstring member comprises a first end securely fastened to the chassis assembly within the ear members and a second end securely fastened to the chassis assembly within the front region. Preferably, the secured first ends of the drawstring members are secured within the grasping zones of each ear member.

In a preferred alternative of the above embodiment, a waist fit member of the same material types as the drawstring members is positioned adjacent to the end edge within the rear region. More specifically, the waist fit member is connected to each of the drawstring members and is more preferably connected to the first ends of each drawstring member.

In a particularly preferred embodiment of the invention herein the absorbent article comprises a first surface and a second surface attached to the first surface. The attached first and second surfaces comprise a front region, a crotch region adjacent to the front region on one side of the crotch region, a rear region adjacent to the crotch region on an opposite side of the crotch region, a longitudinal axis, a transverse axis, a pair of end edges and a pair of longitudinal edges. The embodiment further comprises a pair of drawstring chambers. Each chamber is positioned adjacent to and extends along a longitudinal edge. Each chamber comprises a closed end in the front region and an open end in the rear region, and a drawstring member positioned within a chamber that extends within the chamber from the front region to the rear region. Each drawstring member has a secured first end in the front region and an unsecured second end extending through the opening in the rear region. The drawstring members each comprises a pull portion that extends out from the chamber openings. Additionally, the pull portions of the drawstring members preferably comprise fastening means thereon for securing the absorbent article about a wearer while simultaneously providing tension about the legs through the drawstring members.

### BRIEF DESCRIPTION OF THE DRAWINGS

While the specification concludes with claims particularly pointing out and distinctly claiming the subject matter which is regarded as forming the present invention, it is believed that the invention will be better understood from the following descriptions which are taken in conjunction with the accompanying drawings in which like designations are used to designate substantially identical elements, and in which:
Figure 1 is a plan view of an absorbent article of the present invention having portions cut-away to reveal underlying structure, the outer surface of the diaper facing the viewer;
Figure 2 is a partial view the absorbent article of Fig. 1 shown in a fastened condition;
Figure 3A is a perspective view of an alternative embodiment of an absorbent article of the present invention;
Figure 3B is a perspective view of the absorbent article of Figure 3A shown in a fastened condition;
Figure 4A is a cross-sectional view of a drawstring chamber of the present invention;
Figure 4B is a plan view of the drawstring chamber in Figure 4A;
Figure 4C is a plan view of an alternative embodiment of the lines of joining in Fig. 4B;
Figure 4D is a plan view of an alternative embodiment of the lines of joining in Fig. 4B;
Figure 4E is a plan view of an alternative embodiment of the lines of joining in Fig. 4B;
Figure 5 is a plan view of an alternative embodiment of the absorbent article of the present invention;
Figure 6 is a plan view of an alternative embodiment of the absorbent article of the present invention; and
Figure 7 discloses a partial view of portions of the backsheet and the topsheet in Fig. 1 being folded over to create a chamber that houses a drawstring member.

### DETAILED DESCRIPTION OF THE INVENTION

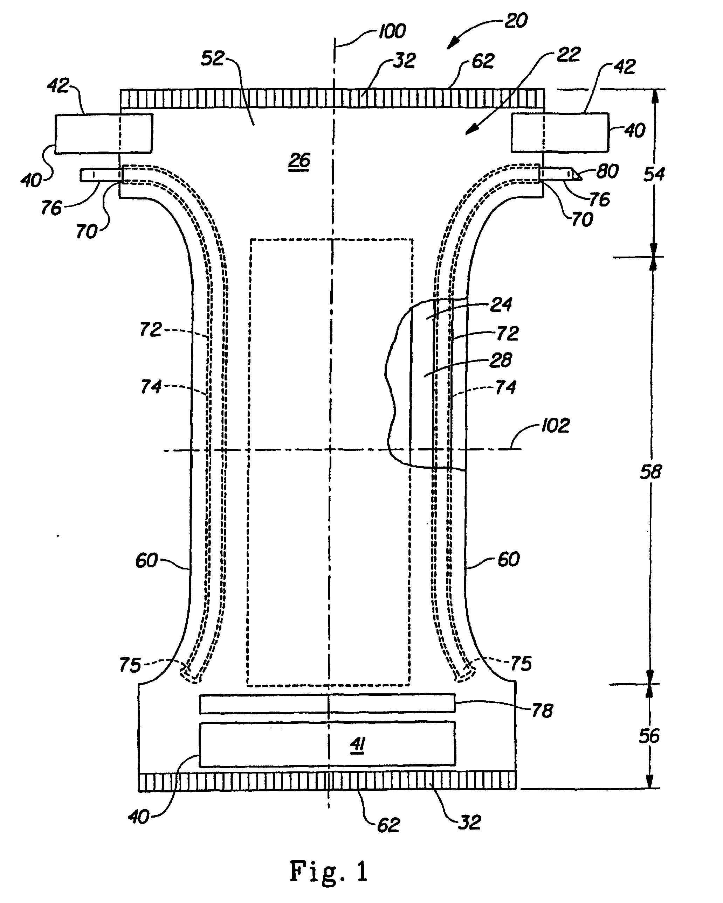

Figure 1 is a plan view of the diaper 20 of the present invention in its flat-out, uncontracted state (i.e., with elastic induced contraction pulled out) with portions of the structure being cut-away to more clearly show the construction of the diaper 20 and with the portion of the diaper 20 which faces away from the wearer, the outer surface 52, facing the viewer. As shown in Figure 1, the diaper 20 comprises a containment assembly or chassis 22 preferably comprising a liquid pervious topsheet 24, a liquid impervious backsheet 26 joined to the topsheet 24, and an absorbent core 28 positioned between the topsheet 24 and the backsheet 26. The diaper preferably further comprises elasticized waistbands 32; a fastening system 40 comprising a pair of first fastening members 42 and a second fastening member 41.

The diaper 20 is shown in Figure 1 to have an outer surface 52 (facing the viewer in Figure 1), an inner surface 50 opposed to the outer surface 52, a front waist region 56, a rear waist region 54 longitudinally opposed to the front waist region 56, a crotch region 58 positioned between the front waist region 56 and the rear waist region 56, and a periphery which is defined by the outer perimeter or edges of the diaper 20 in which the longitudinal edges are designated 60 and the end edges are designated 62. The inner surface 50 (not shown) of the diaper 20 comprises that portion of the diaper 20 which faces toward the wearer's body during use (i.e., the inner surface 50 is generally formed by at least a portion of the topsheet 24 and other components joined to the topsheet 24). The outer surface 52 of the diaper 20 comprises that portion of the diaper 20 which faces away from the wearer's body during use (i.e., the outer surface 52 is generally formed by at least a portion of the backsheet 26 and other components joined to the backsheet 26). As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element. The front waist region 56 and the rear waist region 54 extend from the end edges 62 of the periphery to the crotch region 58.

The diaper 20 also has two centerlines, a longitudinal centerline 100 and a transverse centerline 102. The term "longitudinal", as used herein, refers to a line, axis, or direction in the plane of the diaper 20 that is generally aligned with (e.g., approximately parallel with) a vertical plane which bisects a standing wearer into left and right halves when the diaper 20 is worn. The terms "transverse" and "lateral", as used herein, are interchangeable and refer to a line, axis or direction which lies within the plane of the diaper that is generally perpendicular to the longitudinal direction (which divides the wearer into front and back body halves.)

The containment assembly 22 of the diaper 20 is shown in Figure 1 as comprising the main body (chassis) of the diaper 20. The containment assembly 22 comprises at least an absorbent core 28 and preferably an outer covering layer comprising the topsheet 24 and the backsheet 26. When the absorbent article comprises a separate holder and a liner, the containment assembly 22 generally comprises the holder and the liner (i.e., the containment assembly 22 comprises one or more layers of material to define the holder while the liner comprises an absorbent composite such as a topsheet, a backsheet, and an absorbent core.) For unitary absorbent articles, the containment assembly 22 comprises the main structure of the diaper with other features added to form the composite diaper structure. Thus, the containment assembly 22 for the diaper 20 generally comprises the topsheet 24, the backsheet 26, and the absorbent core 28.

Figure 1 shows a preferred embodiment of the containment assembly 22 in which the topsheet 24 and the backsheet 26 have length and width dimensions generally larger than those of the absorbent core 28. The topsheet 24 and the backsheet 26 extend beyond the edges of the absorbent core 28 to thereby form the periphery of the diaper 20. While the topsheet 24, the backsheet 26, and the absorbent core 28 may be assembled in a variety of well known configurations, preferred diaper configurations are described generally in U.S. Patent 3,860,003 entitled "Contractible Side Portions for Disposable Diaper" which issued to Kenneth B. Buell on January 14, 1975; and U.S. Patent 5,151,092 entitled "Absorbent Article With Dynamic Elastic Waist Feature Having A Predisposed Resilient Flexural Hinge", which issued to Kenneth B. Buell et al on September 29, 1992.

Additionally, Figure 1 discloses an embodiment wherein drawstring chambers 72 (or chambers) extend from the front waist region 56 to the rear waist region 54. These chambers 72 are positioned adjacent to the longitudinal edges 60 and preferably fitted between the topsheet 24 and backsheet 26. In one embodiment, the chambers 72 may comprise materials that are separate from the topsheet 24 and backsheet 26. That is, the chambers 72 may be separate members positioned between the topsheet 24 and backsheet 26. The chambers 72, as separate members herein, may be bonded or attached to the topsheet 24 and backsheet 26. Suitable bonding means herein may include adhesives and/or cohesive bonds known in the art, mechanical bonds, ultrasonic bonds, thermal bonds, and the like. In this embodiment, the chambers 72 should comprise materials that are compatible with the topsheet and backsheet: for example, nonwovens, polypropylene, polyethylene, and any other of the known fibers and synthetic materials used in like absorbent articles.

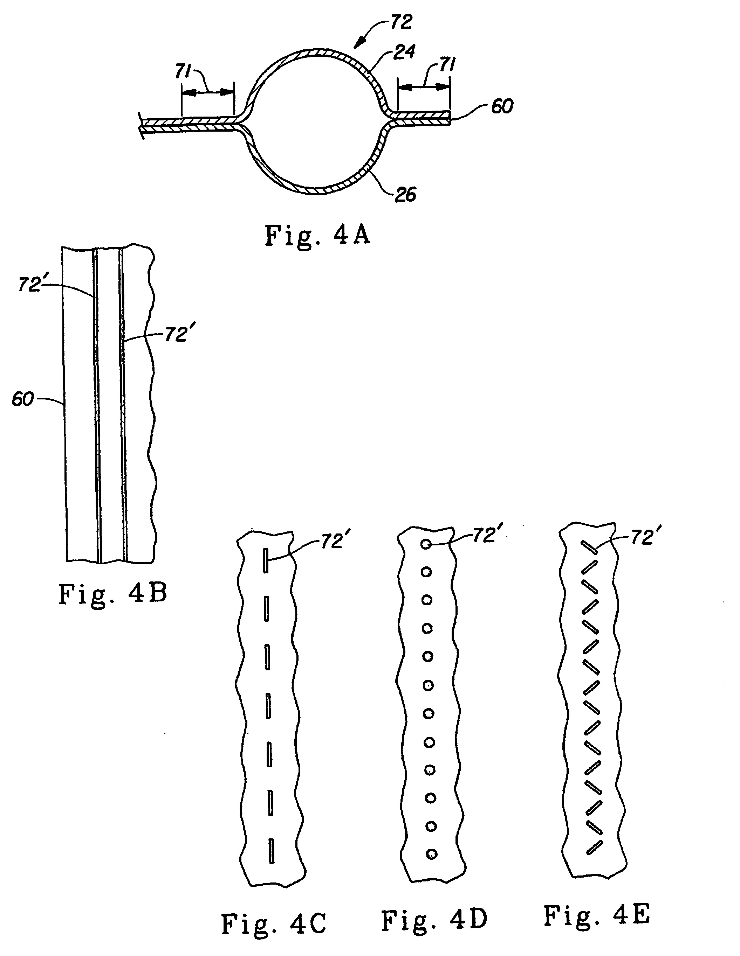

In one preferred embodiment, the chambers 72 are not separate members from the topsheet 24 and backsheet 26. Rather, the topsheet 24 and backsheet 26 together form the chambers 72 herein. This embodiment is preferred because it provides a savings in material usage and unit operations, i.e., fitting and attaching a chamber as a separate member to the diaper containment assembly 22. In this preferred embodiment, the chambers 72 are preferably formed from portions of the topsheet 24 and backsheet 26 brought together (e.g., folded over) and bonded in pre-determined locations to form an enclosure like that represented in Figures 4A and 4B. In Fig. 4A, a cross-section of a chamber 72 is shown in which portions of the topsheet 24 and backsheet 26 that are adjacent to a longitudinal edge 60 have been brought together along two points or regions of joining 71, which when the chamber is completed become two lines of joining 72'. This bonding can occur through any one of the known suitable adhesives used in disposable absorbent articles herein (i.e., for diapers and incontinent devices), but most preferably occurs through bonding (e.g., mechanical bonds, ultrasonic bonds, thermal bonds, and the like) the topsheet 24 and the backsheet 26 together along the lines of joining 72' shown in Fig. 4B. Lines of joining 72' may additionally comprise generally linear arrangements of spaced intermittent bonds as is shown in Figs. 4C, 4D and 4E..

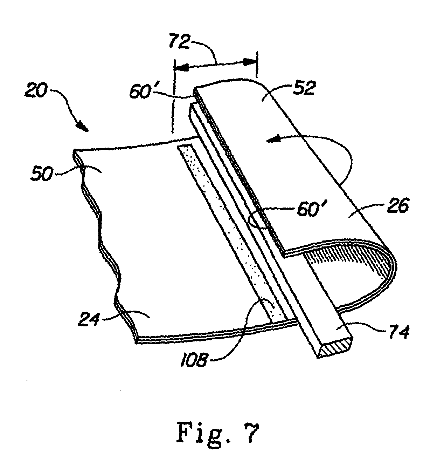

In an alternative embodiment of the chambers 72 herein, the chambers 72 may be formed from a portion of the backsheet 26 which has been folded over onto the topsheet 24, or a portion of the topsheet 24 which has been folded over onto backsheet 26. In another option, Figure 7 discloses a partial view of portions of the backsheet 26 and the topsheet 24 being folded over to create a chamber 74 that houses a drawstring member 74. As is shown, the topsheet 24 and backsheet 26 portions are folded over and at least the end edge 60' is attached to the inner surface 50 of the chassis 22 by a bonding means 108. The bonding means 108 can be one or more of the adhesive and/or cohesive bonds known in the art, mechanical bonds, ultrasonic bonds, thermal bonds, and the like.

In the embodiment shown in Fig. 1 drawstring members 74 are positioned within each chamber 72. Each enclosed chamber 72 has a chamber end opening 70 in the rear waist region 54. The drawstring members 74 have their first ends 75 securely fastened to the chassis 22 within the front waist region 56 and their second ends or pull portions 76 extending outboard of the openings 70 in the rear waist region 54. The drawstring members 74 have fastening elements 80 at the second ends 76. Additionally but not necessarily, the diaper 20 may include one or more receiving elements or members 78 on the outer surface 52 of the backsheet 26, i.e., to receive the fastening elements 80.

The drawstring members 74 herein can be elastic and/or inelastic and are selected from the group of materials consisting of rubber elastics, synthetic rubber elastics, elastomeric foams, structural elastic-like film, elastomeric films, nonwovens, polyethylene's, polypropylenes, laminates foams, highloft nonwovens, string, twine, and braided rope. Suitable structural elastic-like films are disclosed in U.S. Patent No. 5,518,801 issued to Chappell, et al on May 21, 1996.

As is seen in Fig. 1, the drawstring members 74 are attached in the front waist region 56 at their first ends 75. From this point of attachment, the drawstring members 74 are substantially unattached throughout the chambers 72 and preferably the drawstring members 74, except for their attachment at their first ends 75, are not attached anywhere else within the chambers 72 and are therefore free to move within the confines of the chambers 72. The ability of the drawstring members 74 to move freely within the chambers 72 is important so that uniform tension is provided throughout the chambers 72 when the members 74 are pulled. Having uniform tension throughout the chambers 72 is important to ensure proper fit of a diaper 20 about a wearer. Unevenly tensioned chambers 72 may result in uneven or improper fit of a diaper 20 about the legs of a wearer.

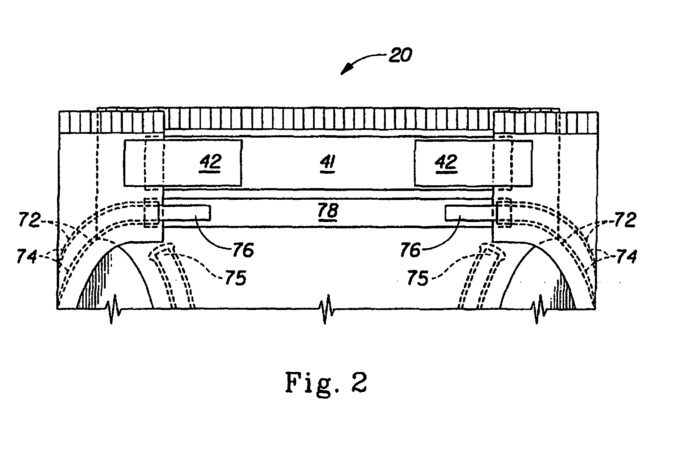

Figure 2 is a partial view of that shows the diaper 20 of Figure 1 in its wear or fastened position. Specifically, Fig. 2 shows the first fastening members or tabs 42 secured to the second fastening member 41 in the front waist region. Additionally, the pull portions 76 of the drawstring members 74 are shown extended beyond the chambers 72 and attached to the receiving member 78. Once the diaper 20 is fitted about a wearer, a diaperer can then adjust the fit about the legs of the wearer by pulling the pull portions 76 to tighten and/or loosen the fit as necessary. The term "diaperer" means herein one who fits a diaper, incontinent device, catamenial pad or other such absorbent articles about the wearer of one these articles. A diaperer and a wearer can be one in the same person. The pull portions 76 herein preferably comprise a fastening means that adheres to either the outer surface 52 of the backsheet 26 or to a receiving member 78. Suitable fastening means herein can be adhesives, hooks and/or loops. Exemplary fastening systems are disclosed in U.S. Patent 4,846,815 entitled "Disposable Diaper Having An Improved Fastening Device" issued to Scripps on July 11, 1989; U.S. Patent 4,894,060 entitled "Disposable Diaper With Improved Hook Fastener Portion" issued to Nestegard on January 16, 1990; U.S. Patent 4,946,527 entitled "Pressure-Sensitive Adhesive Fastener And Method of Making Same" issued to Battrell on August 7, 1990; U.S. Patent 3,848,594 entitled "Tape Fastening System for Disposable Diaper" issued to Buell on November 19, 1974; U.S. Patent B1 4,662,875 entitled "Absorbent Article" issued to Hirotsu et al. on May 5, 1987; and the herein before referenced U.S. Patent Application 07/715,152. Exemplary fastening systems comprising mechanical fastening components (i.e., hooks and loops) are described in U.S. Patent 5,058,247 entitled "Mechanical Fastening Prong" issued to Thomas October 22, 1991; U.S. Patent 4,869,724 entitled "Mechanical Fastening Systems With Adhesive Tape Disposal Means For Disposal of Absorbent Articles" issued to Scripps on September 26, 1989; and U.S. Patent 4,846,815 entitled "Disposable Diaper Having an Improved Fastening Device" issued to Scripps on July 11, 1989. An example of a fastening system having combination mechanical/adhesive fasteners is described in U.S. 4,946,527 entitled "Pressure-Sensitive Adhesive Fastener and Method of Making Same" issued to Battrell on August 7, 1990.

It should be noted herein that the drawstring members 74 are secured to the diaper chassis such that the fastening elements 80 attached to the members 74 are joined to the front region 56. This is important as a wearer will best gain use of the tensioned drawstring members 74 when their tension is exerted through each region; i.e., the front region 56, the crotch region 58 and the rear region 54. Therefore, the best execution of the wear of the embodiments herein exists when the fastening elements 80 of the drawstrings members 74 are attached to the diaper chassis 22 within its front region 56.

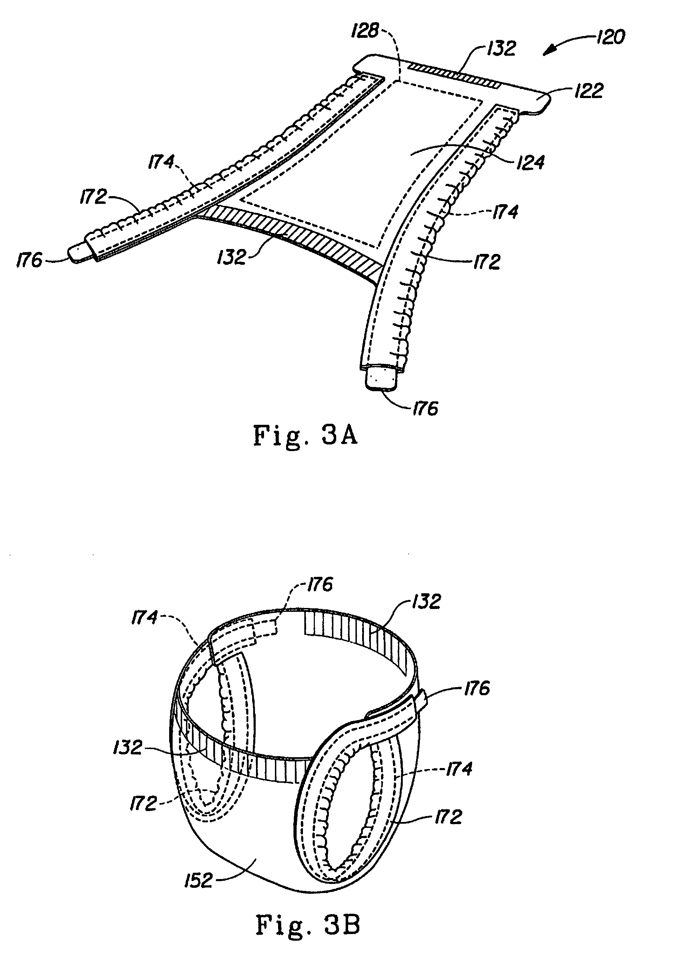

Figures 3A and 3B disclose an alternative embodiment herein whereby the drawstring members operate not only to more securely fit a diaper about a wearer, but also serve as a portion of the fastening system. The fastening system herein is preferably a one element system, e.g., adhesive which attaches to a polyethylene backsheet or hooks which attach to a nonwoven backsheet. This would be critical where manufacturing priorities necessitate a single fastening system whereby only tapes are used to fasten the diaper chassis 22 to a wearer. Fig. 3A shows one embodiment possible for this use. Clearly, one skilled in the art could form any number of designs based upon the inventive concept herein.

Figure 3A shows the diaper 120 in its flat-out uncontracted position with the topsheet 124 facing the viewer. This embodiment comprises an absorbent core 128 fitted between the topsheet 124 and the backsheet 126 (which faces away from the viewer in Fig. 3A). Preferably, the drawstring members 174 herein will be pretensioned such that the diaper chassis 122 will provide curvature compatible with the shape and contour of a wearer. Also preferably, the diaper 120 will comprise waistbands 132 that provide additional stretch about a wearer's torso. Figure 3B shows the diaper 120 of Fig. 3A in it's fitted position. As shown, the pull portions 176 of the diaper 120 will extend out from their chambers 172 and fasten onto either the outer surface 152 of the backsheet 126 or onto a receiving member 178 (not shown). As in the embodiment in Figure 1, the pull portions 176 may comprise any element from the group consisting of adhesives, hooks, and/or loops. Exemplary fastening systems herein are any of those disclosed for Figures 1 and 2.

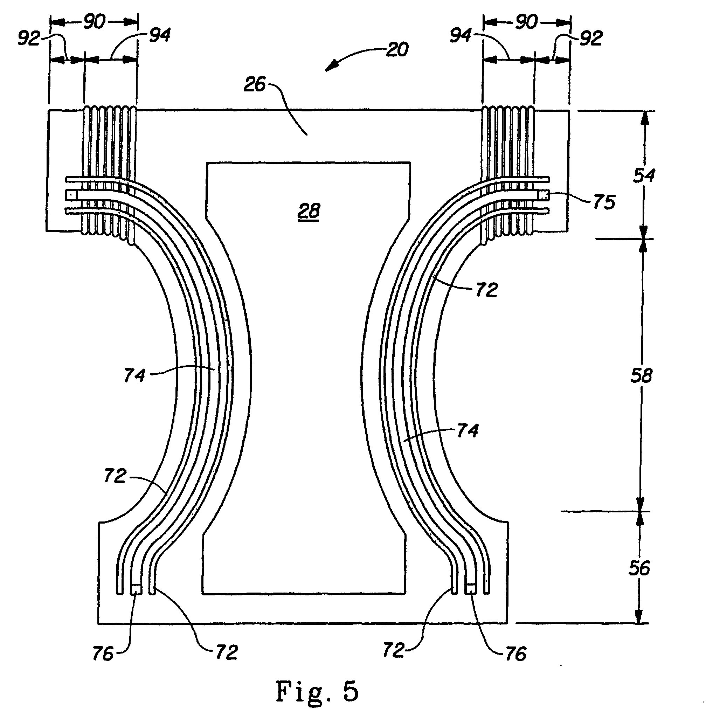

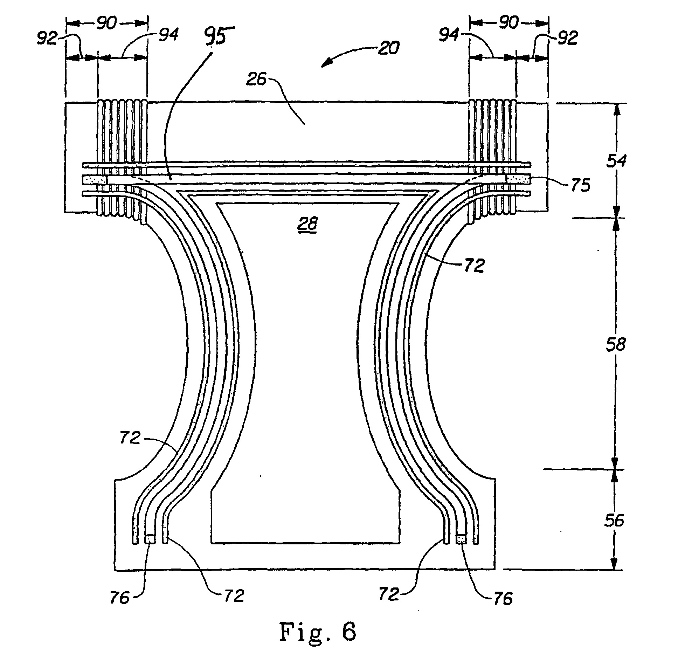

In the embodiments shown in Figures 5 and 6, a diaper 20 comprises a chassis assembly 22 having a topsheet 24, a backsheet 26 having an inner surface 50 and an outer surface 52 joined to the topsheet 24, and an absorbent core 28 located between the topsheet 24 and the backsheet 26. The chassis assembly 22 comprises a front waist region 56, a crotch region 58 adjacent to the front region 56 on one side of the crotch region 58, and a rear waist region 54 adjacent to the crotch region 58 on an opposite side of the crotch region 58. The chassis 22 further comprises a longitudinal axis 100, a transverse axis 102, a pair of end edges 62, and a pair of longitudinal edges 60. Ear members 90 are positioned in the rear region 54 and extend outboard from the longitudinal edges 60 of the absorbent chassis 22. Each ear member 90 comprises a grasping zone 92 and a stretch zone 94. A drawstring chamber 72 is positioned adjacent to each longitudinal edge 60 and extends from the ear members 90 in the front waist region 54 to the rear waist region 56. Each chamber 72 is enclosed. A drawstring member 74 extends through each of the chambers 72. Each drawstring member 74 comprises a first end 75 securely fastened to the chassis assembly 22 within the ear member 90 in the rear region 54 and a second end 76 securely fastened to the chassis assembly 22 within the front waist region 56.

In Figure 5, the diaper 20 is shown with the topsheet 24 removed to reveal the underlying structure, i.e., the absorbent core 28 and backsheet 26. Also, the chamber covering is removed to show drawstring members 74. As shown, the first ends 75 of the drawstrings 74 are securely fastened within the ear members 90. In fact, the first ends 75 are preferably, but not necessarily, fastened within the grasping zones 92 of the ear members 90. This is preferable so that when a diaperer grasps the ear members 90 at the grasping portions 92, the drawstrings 74 will also preferably be directly grasped through the grasping portions 92 and therefore extended to fit about the wearer.

Generally, a diaper 20 of the above embodiment comprising ear members 90 is fitted about a wearer like a conventional diaper, i.e., bringing the front portion 56 through the wearer's legs. Next, a diaperer grasps the ear members 90 at the grasping zones 92 to secure the diaper 20 about a wearer. Preferably, the ear members 90 will comprise fastening tabs 42 with which to fasten a diaper about a wearer. As the diaper 20 is secured to a wearer, i.e., the grasping portions 92 of the ear members 90 are pulled, the drawstrings 74 are put into tension to provide a more secure fit to a wearer about their legs and torso.

The drawstrings 74 and 174 of each of the embodiments herein can be fitted to act alone as extensible leg cuffs or leg cuffs can be included as a separate attachment to the chassis' herein. A more detailed discussion of extensible leg cuffs is provided in latter portions of the specification.

Figure 6 discloses a waist fit member 95 that is positioned adjacent to the end edge 62 within the rear waist region 54. The waist fit member 95 is connected to each drawstring member 74. The waist fit member may be housed within a chamber. The waist fit member 95 is elastic to provide enhanced enclosure about a wearer's waist. Suitable elastics can be selected from the group consisting of rubber elastics, synthetic rubber elastics, elastomeric foams, structural elastic-like film webs and elastomeric films.

The diaper 20 of Figure 6 also preferably comprises a fastening system 40 having first fastening members 42 preferably comprising fastening tabs and a second fastening member 41 that can attach to one-another to form a side closure which maintains the front waist region 54 (Fig. 1) and the rear waist region 56 (Fig. 1) in an overlapping configuration such that lateral tensions are maintained around the circumference of the diaper to maintain the diaper on the wearer. Exemplary fastening systems are disclosed in U.S. Patent 4,846,815 entitled "Disposable Diaper Having An Improved Fastening Device" issued to Scripps on July 11, 1989; U.S. Patent 4,894,060 entitled "Disposable Diaper With Improved Hook Fastener Portion" issued to Nestegard on January 16, 1990; U.S. Patent 4,946,527 entitled "Pressure-Sensitive Adhesive Fastener And Method of Making Same" issued to Battrell on August 7, 1990; U.S. Patent 3,848,594 entitled "Tape Fastening System for Disposable Diaper" issued to Buell on November 19, 1974; U.S. Patent B 1 4,662,875 entitled "Absorbent Article" issued to Hirotsu et al. on May 5, 1987; and the herein before referenced U.S. Patent Application 07/715,152.

Examples of suitable adhesive tape tab fastening systems are disclosed in U.S. Patent 3,848,594 issued to Buell on November 19, 1974; and U.S. Patent 4,662,875 issued to Hirotsu and Robertson on May 5, 1987. Examples of other closure systems, including mechanical closure systems, useful in the present invention, are disclosed in U.S. Patent 4,869,724 issued to Scripps on September 26, 1989; U.S. Patent 4,848,815 issued to Scripps on July 11, 1989; and the two-point fastening system described in U.S. Patent 5,242,436 issued to Weil, Buell, Clear, and Falcone on September 7, 1993. When a two-point fastening system is used, the waist closure members of the waist closure system are preferably longitudinally aligned with the extensible front waist feature 42 and laterally aligned with the elastic strands of the extensible leg cuff 30 to provide an effective closure about both the legs and the waist. As shown throughout the disclosure, the diaper 20 may be provided with a pair of fastening tabs 42 that allow the side panels to be first joined together. The diaperer then brings the containment assembly 22 between the legs of the wearer and joins the assembly 22 to the outer layer of the waist feature. Such a configuration and securing method is more fully described in the above-referenced U.S. Application Serial No. 08/044,562(New, et al.)

The fastening system can comprise any attachment means known in the art including pressure sensitive adhesives, cohesive materials, mechanical fasteners such as hook and loop type fasteners, or any combination of these or any other attachment means known in the art. Exemplary adhesive tape tab fastening systems are disclosed in U.S. Patent 3,848,594 entitled "Tape Fastening System for Disposable Diaper" issued to Buell on November 19, 1974; and U.S. Patent 4,662,875 entitled "Absorbent Article" issued to Hirotsu and Robertson on May 5, 1987. Exemplary fastening systems comprising mechanical fastening components are described in U.S. Patent 5,058,247 entitled "Mechanical Fastening Prong" issued to Thomas October 22, 1991; U.S. Patent 4,869,724 entitled "Mechanical Fastening Systems With Adhesive Tape Disposal Means For Disposal of Absorbent Articles" issued to Scripps on September 26, 1989; and U.S. Patent 4,846,815 entitled "Disposable Diaper Having an Improved Fastening Device" issued to Scripps on July 11, 1989. An example of a fastening system having combination mechanical/adhesive fasteners is described in U.S. 4,946,527 entitled "Pressure-Sensitive Adhesive Fastener and Method of Making Same" issued to Battrell on August 7, 1990.

As discussed, one embodiment of the present invention comprises a hook fastening material preferably comprising a base and a plurality of engaging elements extending from the base. The hook fastening material is intended to engage fibrous elements of a loop fastening material so as to provide a secure fastening device. Thus, the hook fastening material may be manufactured from a wide range of materials. Further, the engaging elements may have any shape such as hooks, "T's", "mushrooms" or any other shape as are well known in the art. Suitable materials include nylon, polyester, polypropylene, or any combination of these materials. Examples of preferred hook fastening materials are available from Aplix of Charlotte, NC under the trade designation 960, 957 and 942. Other preferred hook fastening materials are available from the Minnesota Mining and Manufacturing Company of St. Paul, Minnesota under the trade designations CS200, CS300, MC5 and MC6. Another preferred hook fastening material is described in U.S. Patent 5,058,247 entitled "Mechanical Fastening Prong" issued to Thomas October 22, 1991.

In the fastening systems 40 of the embodiments herein, the second fastening member preferably comprises a fastening element engageable with the hook member. Thus, the second fastening member may be manufactured from a wide range of materials and configurations capable of securely engaging the hook member. For example, the second fastening member may comprise identical complementary elements or distinct complementary elements. As used herein, the term "identical complementary elements" is used to define mechanical fastening systems wherein the engaging elements of the hook member and the second fastening member comprise the same configuration or structure that are interlocking. Examples of such systems are described in Brown et al. U.S. Patent No. 4,322,875 entitled "Two Strip Materials Used For Forming Fasteners" issued on April 16, 1982 and Kellenberger et al. U.S. Patent No. 4,701,179 entitled "Fixed Position Fasteners For Disposable Absorbent Garments" issued on October 20, 1987. The term "distinct complementary elements" is used herein to designate a system wherein the hook member is different from the second fastening member but is engageable therewith.

In one preferred embodiment, the second fastening component comprises a plurality of fiber elements, such as a loop fastening material, that engage the engaging elements of the hook component. The loop fastening material may be manufactured from a wide range of materials to provide fiber elements, preferably being loops. Suitable materials include woven materials, nonwovens, nylons, polyesters, polypropylenes, or any combination of these materials. One suitable loop fastening material is a nonwoven available from Minnesota Mining and Manufacturing Company, St. Paul, Minnesota under the trade designation EBL. A preferred loop fastening material comprises a number of shaped engaging elements projecting from a woven backing such as the commercially available material designated Guilford 18904 available from Guilford Mills of Greensboro, NC. Other suitable loop components are available from the Minnesota Mining and Manufacturing Company of St. Paul, Minnesota under the trade designation EBL. An inexpensive loop fastening material and a method of making the same is described in U.S. Patent 5,032,122, entitled "Loop Fastening Material For Fastening Device and Method of Making Same" issued to Noel et al., July 16, 1991, which application is incorporated herein by reference. Another suitable loop material is described in U.S. Patent 5,326,612 entitled "Nonwoven Female Component for Refastenable Fastening Device and Method of Making the Same" issued to David J.K. Goulait on July 5, 1994. Yet other suitable second fastening components are described in co-pending U.S. Patent Application Serial No. 08/254,814 entitled "Nonwoven Female Component For Refastenable Fastening Device and Method of Making the Same" filed June 6, 1994 in the names of Patrick Jay Alien et al.; U.S. Patent Serial No. 08/287,571 entitled "Nonwoven Female Component For Refastenable Fastening Device" filed August 9, 1994 in the names of Willie F. King et al.; and U.S. Patent Serial No. 08/374,269 entitled "Female Component For Refastenable Fastening Device" filed January 18, 1995 in the names of Carl L. Bergman et al.

The fastening elements may be disposed on either the outer surface or the inner surface of the diaper 20. In a preferred embodiment, however, the fastening elements are disposed on the diaper 20 such that the fasteners do not irritate the wearer's skin. In addition, the fastening elements may either be a discrete separate element affixed to the diaper 20 or a unitary piece of material that is neither divided nor discontinuous with an element of the diaper 20 such as the topsheet 24 or the backsheet 26. While the fastening elements can assume varying sizes and shapes, they preferably comprise one or more separate patches of material joined to the diaper 20 to allow for the best fit for a broad range of wearers.

The topsheet 24 is positioned adjacent the body surface of the absorbent core 28 and is preferably joined thereto and to the backsheet 26 by attachment means (not shown) such as those well known in the art. Suitable attachment means are described with respect to joining the backsheet 26 to the absorbent core 28. As used herein, the term "joined" encompasses configurations whereby an element is directly secured to the other element by affixing the element directly to the other element, and configurations whereby the element is indirectly secured to the other element by affixing the element to an intermediate member(s) which in turn is affixed to the other element. In a preferred embodiment of the present invention, the topsheet 24 and the backsheet 26 are joined directly to each other in the diaper periphery and are indirectly joined together by directly joining them to the absorbent core 28 by the attachment means (not shown).

The topsheet 24 is compliant, soft feeling, and non-irritating to the wearer's skin. Further, the topsheet 24 is liquid pervious permitting liquids (e.g., menses and/or urine) to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. A preferred topsheet is carded and thermally bonded by means well known to those skilled in the fabrics art. A preferred topsheet comprises staple length polypropylene fibers having a denier of about 2.2. As used herein, the term "staple length fibers" refers to those fibers having a length of at least about 15.9 mm (0.625 inches). Preferably, the topsheet has a basis weight from about 18 to about 25 grams per square meter. A suitable topsheet is manufactured by Veratec, Inc., a Division of International Paper Company, of Walpole, Massachusetts under the designation P-8. There are a number of manufacturing techniques which may be used to manufacture the topsheet 24. For example, the topsheet 24 may be a nonwoven web of fibers. When the topsheet comprises a nonwoven web, the web may be spunbonded, carded, wet-laid, meltblown, hydroentangled, combinations of the above, or the like.

A suitable topsheet comprises an apertured formed film. Apertured formed films are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent 3,929,135, entitled "Absorptive Structures Having Tapered Capillaries", which issued to Thompson on December 30, 1975; U.S. Patent 4,324,246 entitled "Disposable Absorbent Article Having A Stain Resistant Topsheet", which issued to Mullane, et al. on April 13, 1982; U.S. Patent 4,342,314 entitled "Resilient Plastic Web Exhibiting Fiber-Like Properties", which issued to Radel. et al. on August 3, 1982; U.S. Patent 4,463,045 entitled "Macroscopically Expanded Three-Dimensional Plastic Web Exhibiting Non-Glossy Visible Surface and Cloth-Like Tactile Impression", which issued to Ahr et al. on July 31, 1984; and U.S. 5,006,394 "Multilayer Polymeric Film" issued to Baird on April 9, 1991.

The apertured topsheet for the present invention is the formed film described in one or more of the above patents and marketed on sanitary napkins by The Procter & Gamble Company of Cincinnati, Ohio as "DRI-WEAVE".

The backsheet 26 is positioned adjacent the garment surface of the absorbent core 28 and is preferably joined thereto by attachment means (not shown) such as those well known in the art. For example, the backsheet 26 may be secured to the absorbent core 28 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola and Tucker on March 4, 1986, and which is incorporated herein by reference. An exemplary attachment means of an open pattern network of filaments comprises several lines of adhesive filaments swirled into a spiral pattern such as is illustrated by the apparatus and methods shown in U.S. Patent 3,911,173 issued to Sprague, Jr. on October 7, 1975; U.S. Patent 4,785,996 issued to Ziecker, et al. on November 22, 1978; and U.S. Patent 4,842,666 issued to Werenicz on June 27, 1989. Alternatively, the attachment means may comprise heat bonds, pressure bonds, heat/pressure bonds, ultrasonic bonds, dynamic mechanical bonds, or any other suitable attachment means or combinations of these attachment means as are known in the art.

The backsheet 26 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body. The backsheet 26 prevents the exudates absorbed and contained in the absorbent core 28 from wetting articles which contact the diaper 20 such as bedsheets and undergarments. The backsheet 26 may thus comprise a woven or nonwoven material, polymeric films such as thermoplastic films of polyethylene or polypropylene, or composite materials such as a film-coated nonwoven material. Preferably, the backsheet is a thermoplastic film having a thickness of from about 0.012 mm (0.5 mil) to about 0.051 mm (2.0 mils). The backsheet preferably comprises a polyethylene blend film of about 0.025 mm (1.0 mil) as is manufactured by Tredegar Corporation of Terre Haute, IN and marketed as P8863.

The absorbent core 28 may be any absorbent means which is capable of absorbing and retaining liquids such as urine and other certain body exudates. The absorbent core 28 has a garment surface, a body surface, side edges, and waist edges. The absorbent core 28 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding, meltblown polymers including coform, cross-linked cellulose fibers, tissue including tissue wraps and tissue laminates, absorbent foams, absorbent sponges, superabsorbent polymers, absorbent gelling materials, or any equivalent material or combinations of materials. The configuration and construction of the absorbent core may also be varied (e.g., the absorbent core may have varying caliper zones, a hydrophilic gradient, a superabsorbent gradient, or lower average density and lower average basis weight acquisition zones; or may comprise one or more layers or structures). The total absorbent capacity of the absorbent core 28 should, however, be compatible with the design loading and the intended use of the diaper 20. Further, the size and absorbent capacity of the absorbent core 28 may be varied to accommodate wearers ranging from infants through adults. Figure 1 shows a preferred embodiment of the diaper 20 having a rectangular-shape absorbent core.

An absorbent structure useful as the absorbent core 28 of the present invention that has achieved wide acceptance and commercial success is described in U.S. Patent 4,610,678 entitled "High-Density Absorbent Structures" issued to Weisman and Goldman on September 9, 1986. U.S. Patent 4,673,402 entitled "Absorbent Articles With Dual-Layered Cores" issued to Weisman, Houghton, and Gellert on June 16, 1987; U.S. Patent 4,888,231 entitled "Absorbent Core Having A Dusting Layer" issued to Angstadt on December 19, 1989; U.S. Patent 5,147,345 entitled "High Efficiency Absorbent Articles For Incontinence Management", issued to Young, LaVon & Taylor on September 15, 1992; U.S. Patent 5,102,597 entitled "Porous, Absorbent, Polymeric Macrostructures and Methods Of Making the Same", issued to Roe, Lahrman and Berg on April 7, 1992; and U.S. Patent 4,834,735, entitled "High Density Absorbent Members Having Lower Density and Lower Basis Weight Acquisition Zones", issued to Alemany and Berg on May 30, 1989; also describe absorbent structures that are useful in the present invention. The absorbent core 28 is preferably the dual-layer absorbent structure described in U.S. Patent 5,234,423 entitled "Absorbent Article With Elastic Waist Feature and Enhanced Absorbency", issued to Alemany and Clear on August 10, 1993.

In a preferred embodiment of the present invention, an acquisition layer(s) may be positioned between the topsheet 24 and the absorbent core 28 and joined to the absorbent core 28. The acquisition layer may serve several functions including improving wicking of exudates over and into the absorbent core. There are several reasons why the improved wicking of exudates is important, including providing a more even distribution of the exudates throughout the absorbent core 28 and allowing the absorbent structure to be made relatively thin. The wicking referred to herein may encompass the transportation of liquids in one, two or all directions (i.e., in the x-y plane and/or in the z-direction). The acquisition layer may be comprised of several different materials including nonwoven or woven webs of synthetic fibers including polyester, polypropylene, or polyethylene; natural fibers including cotton or cellulose; blends of such fibers; or any equivalent materials or combinations of materials. Examples of absorbent structures disclosed herein having an acquisition layer and a topsheet are more fully described in U.S. 4,950,264 issued to Osborn and U.S. Patent Application Serial No. 07/944,764, "Absorbent Article Having Fused Layers", filed October 7, 1992, in the names of Cree, et al. In a preferred embodiment, the acquisition layer may be joined with the topsheet 24 and separate from the inserted absorbent material or absorbent core 28 by any of the conventional means for joining webs together, most preferably by fusion bonds as is more fully described in the above-referenced Cree application.

For all purposes herein, the topsheet 124, backsheet 126, absorbent core 128, and acquisition layers of the Figures 3A and 3B comprise the same materials and are made in the same fashion as those discussed for topsheets 24, backsheets 26, absorbent cores 28, and acquisition layers of all other embodiments herein.

The diapers 20 and 120 may preferably further comprises extensible leg cuffs for providing improved containment of liquids and other body exudates. Each extensible leg cuff may comprise several different embodiments for reducing the leakage of body exudates in the leg regions. (The leg cuff can be and is sometimes also referred to as leg bands, leg flaps, barrier cuffs, or elastic cuffs.) U.S. Patent 3,860,003 entitled "Contractable Side Portions For a Disposable Diaper" issued to Buell on January 14, 1975, describes a disposable diaper which provides a contractible leg opening having a leg flap and one or more elastic members to provide an elasticized leg cuff (gasketing cuff). U.S. Patent 4,909,803 entitled "Disposable Absorbent Article Having Elasticized Flaps" issued to Aziz & Blaney on March 20, 1990, describes a disposable diaper having "stand-up" elasticized flaps (barrier cuffs) to improve the containment of the leg regions. U.S. Patent 4,695,278 entitled "Absorbent Article Having Dual Cuffs" issued to Lawson on September 22, 1987, describes a disposable diaper having dual cuffs including a gasketing cuff and a barrier cuff. U.S. Patent 4,704,115 entitled "Disposable Waist Containment Garment" issued to Buell on November 3, 1987, discloses a disposable diaper or incontinent garment having side-edge-leakage-guard gutters configured to contain free liquids within the garment. U.S. Patent 5,032,120 entitled "Disposable Absorbent Article Having Improved Leg Cuffs" issued to Freeland & Allen on July 16, 1991, discloses an absorbent article having leg cuffs having a relatively low ultimate contact force at relatively high elongation's accomplished, for example, by low contact force differential material. U.S. Patent 5,087,255 entitled "Absorbent Article Having Inflected Barrier Cuffs" issued to Sims on February 11, 1992, discloses an absorbent article having inflected barrier cuffs with the distal edge positioned outboard of the proximal edge in one waist region and inboard in the other to provide better fit about the hips/buttocks.

The diapers 20 and 120 further comprise extensible waist features that provide improved fit and containment. The extensible waist features at least extend longitudinally outwardly from a containment assembly, preferably a respective waist edge of the absorbent core, and generally form at least a portion of the end edge of the diaper 20 or 120. While a disposable diaper of the present invention is constructed with an extensible waist feature disposed in each waist region (an elastic waist feature and an extensible front waist feature), the discussion will focus on diapers having different configurations for each extensible waist feature. At a minimum, it is preferred that the diaper at least have one of the extensible waist features constructed according to the present invention, more preferably at least the elastic waist feature. The waist features can be constructed as a separate element joined to the containment assembly or as an extension of other elements of the diaper (i.e., unitary). The waist features will be described with respect to preferred embodiments in which certain portions or panels comprise an extension of other elements of the diaper such as the backsheet, the topsheet, or both, and other portions or panels comprise a separate element joined to other portions or panels of the waist feature or other panels of the diaper.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. An absorbent article (20) having a chassis assembly (22) comprising a topsheet (24), a backsheet (26) joined to said topsheet (24), said backsheet (26) having an inner surface and an outer surface, an absorbent core (20) located between said topsheet (24) and said backsheet (26), said chassis' assembly (22) having a front region (56), a rear region (54) opposed to said front region (56), a crotch region (58) located between said front region (56) and said rear region (54), a longitudinal axis (100), a transverse axis (102), a pair of end edges (62), a pair of longitudinal edges (60), and fastening means (40) whereby said absorbent article is fastened about a wearer,
a chamber (72) is positioned adjacent each longitudinal edge (60), each said chamber (72) extending from said front region (56) to said rear region (54), each said chamber (72) being substantially enclosed and having an end opening (70) in either said rear region (54) or said front region (56); and
a drawstring member (74) extends through each of said chambers (72), each said drawstring member (74) comprising a first end (75) securely fastened to said chassis assembly (22) within said front region (56) and a second end (76) extending outboard of said end opening (70) in said rear region (54) or each said drawstring member (74) comprising a first end (75) securely fastened to said chassis assembly (22) within said rear region (54) and a second end (76) extending outboard of said opening (70) in said front region (56), said second end (76) of said drawstring member (74) being unattached to said chassis assembly (22) prior to fitting said absorbent article (20) about a wearer,
**characterized in that** said backsheet (26) comprises a receiving element (78) and said second ends (76) of said drawstring member (74) comprise fastening elements (80) such that said drawstring members (74) can be attached to said receiving member (78).

2. The absorbent article of claim 1, further comprising an elastic waist fit member (95) positioned adjacent to said end edge (62) within said rear region (54), said elastic waist fit member (95) being connected to each said drawstring member.

3. The absorbent article (20) of Claim 2 wherein each said first end (75) of said drawstring (74) members is secured within each said grasping zone (92) of each said ear member (90).

4. The absorbent article (20) of Claim 2 or 3 wherein said waist fit members (95) comprise at least one elastic component, one inelastic component, or both, each said elastic component preferably being selected from the group consisting of rubber elastics, synthetic rubber elastics, elastomeric foams, structural elastic-like film webs and elastomeric films, and each said inelastic component preferably being selected from the group consisting of nonwovens, films, laminates, foams, highloft nonwovens, string, twine, and braided rope.

5. The absorbent article (20) of any one of the preceding claims wherein said drawstring members (74) comprise at least one elastic component, at least one inelastic component or both, each said elastic component preferably being selected from the group consisting of rubber elastics, synthetic rubber elastics, elastomeric foams, structural elastic-like film webs and elastomeric films and each said inelastic component preferably being selected from the group consisting of nonwovens, films, laminates, foams, highloft nonwovens, string, twine, and braided rope.

6. The absorbent article (20) of any one of the preceding claims wherein each said chamber (74) is formed by a portion of said topsheet (24), said backsheet (26) or both.

7. The absorbent article (20) of any one of the receding claims wherein said second ends (76) of each said drawstring member (74) comprises at least one fastening element (80), at least one receiving element (78) or both, said fastening elements preferably being selected from the group consisting of hooks, loops, and adhesive tapes, said receiving elements preferably being selected from the group consisting of hooks, loops, nonwovens and fastening surfaces.

## Patentansprüche

1. Absorbierender Artikel (20) mit einer Grundkörpereinheit (22) umfassend eine Oberschicht (24), eine mit der Oberschicht (24) verbundene Unterschicht (26), wobei die Unterschicht (26) eine innere Oberfläche und eine äußere Oberfläche aufweist, einen absorbierenden Kern (20), der zwischen der Oberschicht (24) und der Unterschicht (26) angeordnet ist, wobei die Grundkörpereinheit (22) aufweist eine vordere Region (56), eine hintere Region (54), die der vorderen Region (56) gegenüber liegt, eine zwischen der vorderen Region (56) und der hinteren Region (54) angeordnete Schrittregion (58), eine Längsachse (100), eine Querachse (102), ein Paar Stirnränder (62), ein Paar Längsränder (60) und ein Befestigungsmittel (40), mit welchem der absorbierende Artikel um einen Träger herum festgelegt ist,
wobei eine Kammer (72) angrenzend an jeden Längsrand (60) positioniert ist, wobei sich jede Kammer (72) von der vorderen Region (56) zu der hinteren Region (54) erstreckt, wobei jede Kammer (72) im wesentlichen umschlossen ist und eine Endöffnung (70) entweder in der hinteren Region (54) oder der vorderen Region (56) hat; und
wobei sich ein Zugbandelement (74) durch jede der Kammern (72) erstreckt, wobei jedes Zugbandelement (74) aufweist ein erstes Ende (75), das mit der Grundkörpereinheit (22) innerhalb der vorderen Region (56) fest verbunden ist, und ein zweites Ende (76), das sich aus der Endöffnung (70) in der hinteren Region (54) nach außen erstreckt, oder jedes Zugbandelement (74) ein erstes Ende (75), das mit der Grundkörpereinheit (22) innerhalb der hinteren Region (54) fest verbunden ist, und ein zweites Ende (76), das sich aus der Öffnung (70) in der vorderen Region (56) nach außen erstreckt, wobei das zweite Ende (76) des Zugbandelements (74) vor dem Anlegen des absorbierenden Artikels (20) um einen Träger herum an der Grundkörpereinheit lose angeordnet ist,
**dadurch gekennzeichnet, daß** die Unterschicht (26) ein Aufnahmeelement (78) aufweist und die zweiten Enden (76) des Zugbandelement (74) Befestigungselemente (80) aufweisen, derart, daß die Zugbandelemente (74) an dem Aufnahmeelement (78) angebracht werden können.

2. Absorbierender Artikel nach Anspruch 1, ferner mit einem elastischen Taillen-Passelement (95), das angrenzend an den Stirnrand (62) innerhalb der hinteren Region (54) positioniert ist, wobei das elastische Taillen-Passelement mit jedem Zugbandelement verbunden ist.

3. Absorbierender Artikel (20) nach Anspruch 2, in welchem das erste Ende (75) der Zugbandelemente (74) innerhalb jeder Anfaßzone jedes Flügelelements (90) festgelegt ist.

4. Absorbierender Artikel (20) nach Anspruch 2 oder 3, in welchem die Taillen-Passelemente (95) aufweisen wenigstens eine elastische Komponente, eine unelastische Komponente oder beide, wobei die elastische Komponente vorzugsweise ausgewählt ist aus der Gruppe bestehend aus Gummis, synthetischen Gummis, elastomeren Schäumen, strukturell elastikartigen Filmbahnen und elastomeren Filmen, und die unelastische Komponente vorzugsweise ausgewählt ist aus der Gruppe bestehend aus Vliesstoffen, Filmen, Laminaten, Schäumen, Highloft-Vliesstoffen, Schnur, Zwirn und geflochtenes Band.

5. Absorbierender Artikel (20) nach einem der vorstehenden Ansprüche, in welchem die Zugband-Elemente (74) umfassen wenigstens eine elastische Komponente, wenigstens eine unelastische Komponente oder beide, wobei die elastische Komponente vorzugsweise ausgewählt ist aus der Gruppe bestehend aus Gummis, synthetischen Gummis, elastomeren Schäumen, strukturell elastikartigen Filmbahnen und elastomeren Filmen. und jede unelastische Komponente vorzugsweise ausgewählt ist aus der Gruppe bestehend aus Vliesstoffen, Filmen, Laminaten, Schäumen Highloft-Vliesstoffen, Schnüre, Zwirn und geflochtenes Band.

6. Absorbierender Artikel (20) nach einem der vorstehenden Ansprüche, in welchem die Kammer (74) durch einen Bereich der Oberschicht (24), der Unterschicht (26) oder beiden gebildet ist.

7. Absorbierender Artikel (20) nach einem der vorstehenden Ansprüche, in welchem die zweiten Enden (76) jedes Zugband-Elements (74) aufweisen wenigstens ein Befestigungselement (80), wenigstens ein Aufnahmeelement (78) oder beide,
wobei die Befestigungselemente vorzugsweise ausgewählt sind aus der Gruppe bestehend aus Haken, Schlingen und Haftstreifen, wobei die Aufnahmeelemente vorzugsweise ausgewählt sind aus der Gruppe bestehend aus Haken, Schlingen, Vliesstoffen und Befestigungsoberflächen.

## Revendications

1. Article absorbant (20) comportant un ensemble-châssis (22) comprenant une feuille de dessus (24), une feuille de fond (26) réunie à ladite feuille de dessus (24), ladite feuille de fond (26) présentant une surface intérieure et une surface extérieure, une âme absorbante (28) située entre ladite feuille de dessus (24) et ladite feuille de fond (26), ledit ensemble-châssis (22) présentant une région avant (56), une région arrière (54) opposée à ladite région avant (56), une région d'entrejambe (58) situés entre ladite région avant (56) et ladite région arrière (54), un axe longitudinal (100), un axe transversal (102), une paire de bords d'extrémité (62), une paire de bords longitudinaux (60) et des moyens d'attache (40) faisant en sorte que ledit article absorbant est fixé autour d'un porteur ;
une chambre (72) est placée en position adjacente à chaque bord longitudinal (60), chaque dite chambre (72) s'étendant de ladite région avant (56) à ladite région arrière (54), chaque dite chambre (72) étant essentiellement fermée et présentant une ouverture d'extrémité (70), soit dans ladite région arrière (54), soit dans ladite région avant (56) ; et
un élément (74) formant cordon de serrage s'étend à travers chacune desdites chambres (72), chaque dit élément (74) formant cordon de serrage comprenant une première extrémité (75) fixée de manière sûre audit ensemble-châssis (22) à l'intérieur de ladite région avant (56) et une deuxième extrémité (76) s'étendant à l'extérieur de ladite ouverture d'extrémité (70) ménagée dans ladite région arrière (54), ou bien chaque dit élément (74) formant cordon de serrage comprenant une première extrémité (75) fixée de manière sûre audit ensemble formant châssis (22) à l'intérieur de ladite région arrière (54), et une deuxième extrémité (76) s'étendant à l'extérieur de ladite ouverture d'extrémité (70) ménagée dans ladite région avant (56), ladite deuxième extrémité (76) dudit élément (74) formant cordon de serrage n'étant pas fixée audit ensemble-châssis (22) avant l'ajustement dudit article absorbant (20) autour du porteur,
**caractérisé en ce que** ladite feuille de fond (26) comprend un élément de réception (78), et lesdites deuxièmes extrémités (76) dudit élément (74) formant cordon de serrage comprennent des éléments d'attache (80) afin que lesdits éléments (74) formant cordons de serrage puissent être fixés audit élément de réception (78).

2. Article absorbant selon la revendication 1, comprenant, en outre, un élément élastique (95) d'ajustement de ceinture placé près dudit bord d'extrémité (62) à l'intérieur de ladite région arrière (54), ledit élément élastique (95) d'ajustement de ceinture étant relié à chaque élément formant cordon de serrage.

3. Article absorbant (20) selon la revendication 2, dans lequel chaque dite première extrémité (75) desdits éléments (74) formant cordons de serrage est assujettie à l'intérieur de chaque zone de préhension (92) de chaque élément formant oreille (90).

4. Article absorbant (20) selon la revendication 2 ou 3, dans lequel lesdits éléments (95) d'ajustement de ceinture comprennent au moins un composant élastique, un composant non élastique, ou un composant élastique et un composant non élastique, chaque dit composant élastique étant choisi, de préférence, dans le groupe constitué par les élastiques en caoutchouc, les élastiques en caoutchouc synthétique, les mousses élastomères, les nappes à film de structure de type élastique et les films élastomères, et chaque dit composant non élastique étant choisi, de préférence, dans le groupe constitué par les non tissés, les films, les stratifiés, les mousses, les non tissés à bouffant élevé, les cordons, les ficelles, et les cordes tressées.

5. Article absorbant (20) selon l'une quelconque des revendications précédentes, dans lequel lesdits éléments (74) formant cordons de serrage comprennent au moins un composant élastique, au moins un composant non élastique, ou un composant élastique et un composant non élastique, chaque dit composant élastique étant choisi, de préférence, dans le groupe constitué par les élastiques en caoutchouc, les élastiques en caoutchouc synthétique, les mousses élastomères, les nappes à film de structure de type élastique et les films élastomères, et chaque dit composant non élastique étant choisi, de préférence, dans le groupe constitué par les non tissés, les films, les stratifiés, les mousses, les non tissés à bouffant élevé, les cordons, les ficelles, et les cordes tressées.

6. Article absorbant (20) selon l'une quelconque des revendications précédentes, dans lequel chaque dite chambre (72) est formée par une partie de ladite feuille de dessus (24), de ladite feuille de fond (26) ou de ces deux dernières.

7. Article absorbant (20) selon l'une quelconque des revendications précédentes, dans lequel lesdites deuxièmes extrémités (76) de chaque élément (74) formant cordon de serrage comprennent au moins un élément d'attache (80), au moins un élément de réception (78), ou les deux, lesdits éléments d'attache étant choisis, de préférence, dans le groupe constitué par les crochets, les boucles et les rubans adhésifs, lesdits éléments de réception étant choisis, de préférence, dans le groupe constitué par les crochets, les boucles, les non tissés et les surfaces d'attache.
